# FASCICULE DE BREVET EUROPEEN

(11) **EP 2 563 432 B1**
(45) Date de publication et mention de la délivrance du brevet: **01.09.2021**
(21) Numéro de dépôt: 11723531.7
(22) Date de dépôt: 28.04.2011
(51) Int. Cl.: A61M 5/31, A61M 5/32

(54) **DISPOSITIF DE SECURITE POUR SERINGUE D'INJECTION PRE-REMPLIE**
SICHERHEITSVORRICHTUNG FÜR EINE VORGEFÜLLTE INJEKTIONSSPRITZE
SAFETY DEVICE FOR A PRE-FILLED INJECTION SYRINGE

(30) Priorité: 28.04.2010 FR 1053280
(43) Date de publication de la demande: 06.03.2013
(73) Titulaire: NEMERA LA VERPILLIERE, 38290 La Verpillière (FR)
(72) Inventeur: DUGAND, Pascal, 38780 Estrablin (FR); LANZI, Sylvain, 38850 Chirens (FR)
(74) Mandataire: Vallée-Thiollier, Clémence-Olivia Laure Marie
(86) Numéro de dépôt international: PCT/FR2011/050975
(87) Numéro de publication internationale: WO 2011/135269

(56) Documents cités:
- WO-A2-03/077977
- DE-A1- 19 613 035
- FR-A1- 2 842 112
- US-A1- 2006 036 216
- US-B1- 6 319 233

## Description

La présente invention concerne le domaine de l'injection de produits tels que des médicaments. Il concerne plus particulièrement le domaine des dispositifs de sécurité pour seringue d'injection pré-remplie.

On connaît déjà un dispositif de sécurité pour seringue d'injection, muni d'un fourreau protecteur déclenché en fin d'injection afin de recouvrir l'aiguille d'injection, et ainsi éviter des contaminations. Un exemple de dispositif est décrit dans FR2922112, WO03/077977 et US2006/0036216.

Généralement, un tel dispositif comprend des ailettes, ou encore une collerette, servant de surface d'appui pour les doigts de l'utilisateur qui procède à l'injection.

Une difficulté réside dans le fait que ces ailettes de préhension peuvent être trop petites pour des personnes qui souffrent de douleurs inflammatoires, articulaires ou qui ont des difficultés à manipuler ou à appliquer des efforts suffisants sur des produits de petite taille, par exemple des personnes qui souffrent de polyarthrite rhumatoïde.

On connaît par ailleurs du document DE19613035 un appui-doigt configuré pour coulisser le long d'une seringue en verre. La présente invention a notamment pour but de fournir un dispositif de sécurité dont la manipulation est plus aisée, en particulier pour des utilisateurs rencontrant des difficultés de préhension du dispositif.

A cet effet, l'invention a pour objet un dispositif de sécurité pour seringue d'injection, comprenant :
- un support de seringue,
- un fourreau de protection d'une aiguille d'injection portée par la seringue, le fourreau étant monté coulissant par rapport au support de seringue entre une position d'injection, dans laquelle l'aiguille est découverte, et une position de protection, dans laquelle l'aiguille est recouverte, le fourreau comportant une surface de préhension du dispositif par un utilisateur, appelée surface de préhension initiale,
- un ressort de rappel du fourreau en position de protection,
le fourreau comportant en outre un organe d'extension de la surface de préhension initiale, rapporté sur le fourreau, de façon à présenter une surface de préhension plus grande, dite surface de préhension agrandie.

Ainsi, on propose de rapporter sur le fourreau un élément permettant d'offrir une préhension plus facile du dispositif. Dans certains cas, il peut être avantageux de rapporter un élément supplémentaire sur le fourreau pour agrandir une surface de préhension initiale plutôt que de réaliser directement une grande surface de préhension sur le fourreau. En effet, comme il a été constaté que seuls certains utilisateurs ont besoin d'une surface agrandie, on propose ici de réaliser un dispositif standard, destiné à tout type d'utilisateurs, tout en laissant la possibilité d'adapter le dispositif de sécurité à certains utilisateurs, en ajoutant sur le dispositif l'organe d'extension. Il en résulte que la fabrication est simplifiée, puisque l'on conserve un support de seringue et un fourreau identiques à ceux utilisés précédemment, donc un même moule, tout en offrant la garantie que des personnes qui éprouvent des difficultés de manipulation pourront utiliser le dispositif sans difficulté, en laissant la possibilité de rapporter l'organe d'extension sur le fourreau. En outre, on peut ainsi conserver une surface de préhension relativement petite pour les utilisateurs habituels, la surface initiale ménagée sur le fourreau, ce qui permet de réduire l'encombrement général du dispositif de sécurité et donc de gagner de la place pour le stockage et le transport des dispositifs.

On notera que la seringue d'injection est de préférence pré-remplie et est généralement une seringue en verre, de taille et de forme standards, par exemple une seringue de 0,5 ou 1 ml (millilitre). La seringue est de forme tubulaire, présentant une aiguille d'injection sur son extrémité distale, éventuellement montée sur un embout de fixation souvent appelé « luer lock », et présentant une collerette sur son extrémité proximale, venue de matière avec le corps de seringue. La collerette de la seringue permet en particulier de fixer la seringue sur le support de seringue, par exemple par encliquetage. On notera par ailleurs que le support de seringue est généralement réalisé en matière plastique, ce qui offre l'avantage de réaliser plus facilement des parties fonctionnelles sur le support que sur la seringue qui est en verre. Par exemple, le support de seringue peut porter des ergots d'encliquetage de la collerette de la seringue, des éléments de guidage du fourreau jusqu'à sa position de protection, des éléments de verrouillage du dispositif en position de protection et/ou des éléments escamotables d'immobilisation du dispositif en position d'injection. On notera également que le fourreau est généralement une pièce en matière plastique qui assure plusieurs fonctions. Le fourreau peut comporter l'un ou plusieurs des éléments suivants : des moyens escamotables de compression du ressort pour maintenir le dispositif en position d'injection, des moyens de guidage du fourreau lors de son déplacement jusqu'à la position de protection, des moyens d'immobilisation du fourreau en position de protection, une partie tubulaire pour recouvrir l'aiguille et pour empêcher le doigt d'un utilisateur d'accéder à l'aiguille une fois le dispositif en position de protection.

La surface de préhension agrandie est de préférence une surface qui recouvre la surface de préhension initiale et qui s'étend selon un plan sensiblement perpendiculaire à l'axe du dispositif.

Grâce à l'organe d'extension présenté ci-dessus, les inventeurs proposent de faire un pas supplémentaire dans la standardisation des procédés de fabrication ; non seulement on peut utiliser une seringue standard en verre, mais on peut également utiliser un fourreau standard, destiné à tout type d'utilisateurs, en rapportant ensuite éventuellement l'organe d'extension.

Le dispositif de sécurité peut en outre comporter l'une ou plusieurs des caractéristiques suivantes, prises seules ou en combinaison.
- L'organe d'extension comporte une jupe annulaire faisant saillie de la surface de préhension agrandie. Cette jupe annulaire est de préférence disposée du côté distal par rapport à la surface de préhension agrandie. Elle est coaxiale avec le fourreau, en étant disposée autour du fourreau, et peut assurer une fonction de centrage de l'organe d'extension sur le fourreau, en maintenant la surface de préhension agrandie en position dans l'axe du fourreau et en évitant ainsi qu'elle se désaxe en raison d'un mouvement asymétrique de l'utilisateur sur un côté de la surface de préhension. La jupe annulaire peut également servir de support pour une étiquette d'information relative au médicament qui se trouve dans la seringue. En effet, l'avantage de la jupe annulaire est qu'elle ne présente pas de relief, à la différence d'un fourreau qui peut porter par exemple des clips d'immobilisation du fourreau en position de protection, peu pratiques pour apposer une étiquette. Aussi, la jupe annulaire permet de présenter une surface relativement grande sans relief, idéale pour supporter une étiquette.
- Le ressort est dans une configuration comprimée lorsque le fourreau est en position d'injection, et la jupe annulaire est une jupe de masquage du ressort en configuration comprimée. Souvent, le fourreau et le support de seringue sont réalisés dans un matériau transparent ou translucide, pour permettre à l'utilisateur de voir le contenu de la seringue qui se trouve à l'intérieur du dispositif. Il en résulte que l'utilisateur voit généralement le ressort du dispositif de sécurité. Or, le ressort est souvent une pièce en métal, qui peut donner des a priori négatifs aux utilisateurs quant à la stérilité du produit injecté ou à la propreté du système. En effet, bien que le ressort ne soit jamais en contact avec le produit injecté, il donne une impression générale de dispositif technique, "non hygiénique". Le fait de le cacher, grâce à une jupe annulaire opaque, donne à l'utilisateur l'impression que le dispositif de sécurité est plus stérile ou plus propre.

- Le fourreau comporte des moyens d'immobilisation du fourreau en position de protection, de préférence des moyens d'encliquetage, et la jupe annulaire recouvre ces moyens d'immobilisation du fourreau. Ainsi la jupe annulaire permet d'une part de masquer les moyens d'immobilisation, ce qui est plus esthétique, et d'autre part de rendre ces moyens plus difficilement accessibles par l'utilisateur, ce qui empêche de réutiliser le dispositif de sécurité une fois qu'il est en position de protection. Cet avantage est particulièrement intéressant pour empêcher l'utilisation de dispositifs usagés et/ou pour réduire le risque de blessure. En outre, la jupe présente ainsi une surface encore plus grande pour servir de support d'étiquette.
- La jupe annulaire s'étend dans la direction longitudinale jusqu'à l'extrémité distale du dispositif, de façon à recouvrir toute l'aiguille de la seringue lorsque le fourreau est en position de protection. Outre le fait que la jupe annulaire permet de présenter une surface très grande pour supporter une étiquette, cela peut permettre à l'organe d'extension d'assurer la fonction de recouvrement de l'aiguille en position d'injection et ainsi de simplifier le reste du fourreau en l'allégeant de cette fonctionnalité, ce qui peut être particulièrement intéressant pour permettre un démoulage simple du reste du fourreau. En effet, la partie du fourreau qui recouvre l'aiguille en position de protection a souvent besoin de présenter une diminution de diamètre pour empêcher l'introduction d'un doigt après instauration de la position de sécurité. Cette diminution de diamètre rend difficile le démoulage du fourreau. Le fait de réaliser un organe d'extension qui recouvre toute l'aiguille permet de réduire la longueur du fourreau initial et donc de ne pas avoir à réaliser cette diminution de diamètre, donc de le rendre plus facile à démouler.
- La jupe annulaire comprend une partie tubulaire principale et une extrémité distale dont le diamètre diminue par rapport à celui de la partie tubulaire principale. En d'autres termes, l'extrémité distale de la partie tubulaire est de forme tronconique.
- La surface de préhension agrandie présente deux extrémités radiales opposées, ces extrémités étant incurvées vers l'extrémité distale du dispositif. Les extrémités incurvées permettent une préhension plus aisée pour les utilisateurs qui ont des difficultés, en évitant notamment que le dispositif leur échappe de la main.
- La surface de préhension agrandie présente des reliefs antidérapants, par exemple des nervures.
- La surface de préhension agrandie est réalisée ou recouverte complètement ou partiellement d'un matériau plus souple que le reste du dispositif de sécurité. Ce matériau souple assure plus de confort dans la préhension du dispositif de sécurité, tout particulièrement pour les utilisateurs ayant une peau qui pourrait être sensible à des arêtes dures.
- Le dispositif comporte des moyens d'encliquetage de l'organe d'extension sur le fourreau, portés par l'organe d'extension et/ou le fourreau. Ils sont en particulier portés par la paroi initiale de préhension du dispositif.
- La surface de préhension agrandie présente deux extrémités radiales opposées, connectées par deux bords présentant chacun un méplat. Ces méplats permettent d'orienter le dispositif pendant le procédé de fabrication du dispositif, et facilitent en outre son stockage, empêchant que le dispositif ne "roule".
- Le fourreau et le support sont réalisés dans un matériau translucide ou transparent, et l'organe d'extension est réalisé dans un matériau opaque. Ainsi, l'organe d'extension permet de cacher certains éléments. En outre, il peut servir de moyen d'indication du type de médicament qui se trouve dans la seringue. Par exemple, il est réalisé dans une couleur indiquant la nature du médicament qui se trouve dans la seringue, ou encore la contenance ou le taux de remplissage de la seringue.
- L'organe d'extension est rapporté par l'extrémité distale du dispositif. Ainsi, l'utilisateur ne risque pas de démonter l'organe d'extension du fourreau lorsqu'il injecte le produit, c'est-à-dire lorsqu'il appuie sur l'organe d'extension en exerçant une force dirigée vers l'extrémité proximale du dispositif.
- Le dispositif comprend un manchon d'inviolabilité, fixé sur le dispositif par un lien cassable, de préférence venu de moulage avec l'organe d'extension. On peut ainsi s'assurer que le dispositif n'a pas été ouvert avant injection.

L'invention a également pour objet un ensemble de deux dispositifs tels que décrits ci-dessus, présentant chacun un support de seringue et un fourreau identiques et un organe d'extension de la surface de préhension différent d'un dispositif à l'autre. Par exemple, l'organe d'extension présente une forme, une couleur et/ou un matériau différents, ce qui permet de distinguer plusieurs types de dispositifs de sécurité, de seringues ou de médicaments. Éventuellement l'un des dispositifs comprend l'organe d'extension alors que l'autre n'en comporte pas du tout.

L'invention a en outre pour objet un ensemble d'un dispositif tel que décrit ci-dessus et d'une seringue pré-remplie. La seringue est de préférence en verre, munie d'une collerette venue de matière avec le corps de seringue. La collerette de la seringue est un élément distinct de la surface de préhension initiale et de la surface de préhension agrandie.

L'invention a par ailleurs pour objet un procédé d'assemblage d'un dispositif tel que décrit ci-dessus, au cours duquel on assemble tout d'abord une partie du fourreau et le support, puis l'on rapporte l'organe d'extension sur cette partie du fourreau, de préférence par l'extrémité distale du fourreau.

Ce procédé d'assemblage est de préférence suivi d'une étape d'assemblage d'une seringue pré-remplie dans le dispositif de sécurité.

L'invention sera mieux comprise à la lecture de la description qui va suivre, donnée uniquement à titre d'exemple et faite en se référant aux dessins dans lesquels :
- les figures 1a et un 1b sont des vues en coupe longitudinale de l'ensemble d'un dispositif de sécurité et d'une seringue selon un premier mode de réalisation, respectivement en position d'injection et en position de protection,
- les figures 2a et 2b sont des vues en perspective, respectivement de dessus et de dessous, d'une partie de l'ensemble de la figure 1a,
- la figure 3 est une vue en perspective d'un organe d'extension pour un dispositif de sécurité selon un deuxième mode de réalisation,
- la figure 4 est une vue en perspective d'un organe d'extension pour un dispositif de sécurité selon un troisième mode de réalisation,
- la figure 5 est une vue en coupe longitudinale d'un fourreau d'un dispositif de sécurité selon un quatrième mode de réalisation,
- la figure 6 est une vue en perspective de l'ensemble d'un dispositif de sécurité et d'une seringue selon un cinquième mode de réalisation,
- la figure 7 est une vue en perspective de l'ensemble d'un dispositif de sécurité et d'une seringue selon un sixième mode de réalisation,
- la figure 8 est une vue similaire à celle la figure 1a d'un ensemble selon un septième mode de réalisation,
- la figure 9 est une vue similaire à celle la figure 1a d'un ensemble selon un huitième mode de réalisation, et
- la figure 10 est une vue similaire à celle de la figure 1a d'un ensemble selon un neuvième de réalisation.

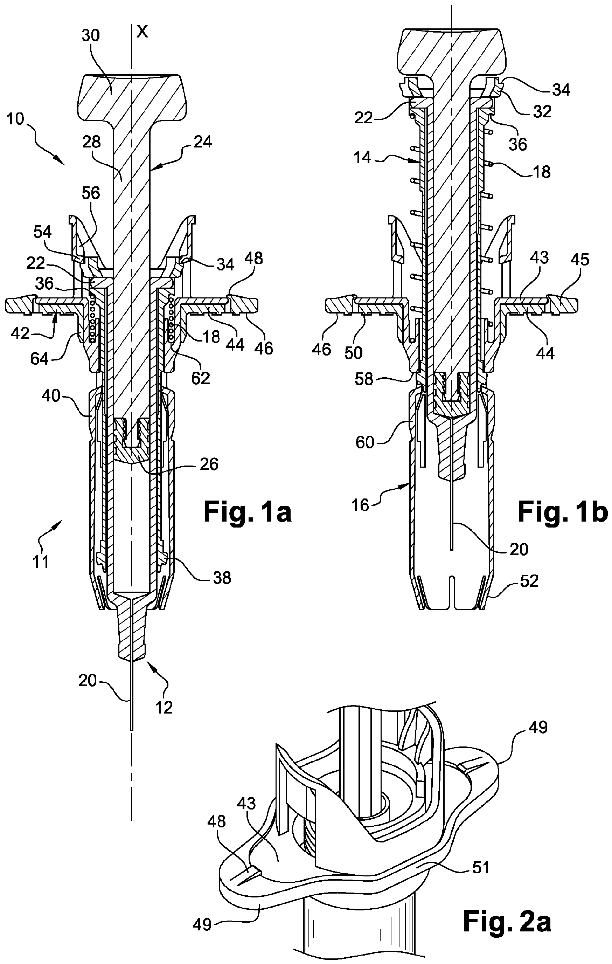

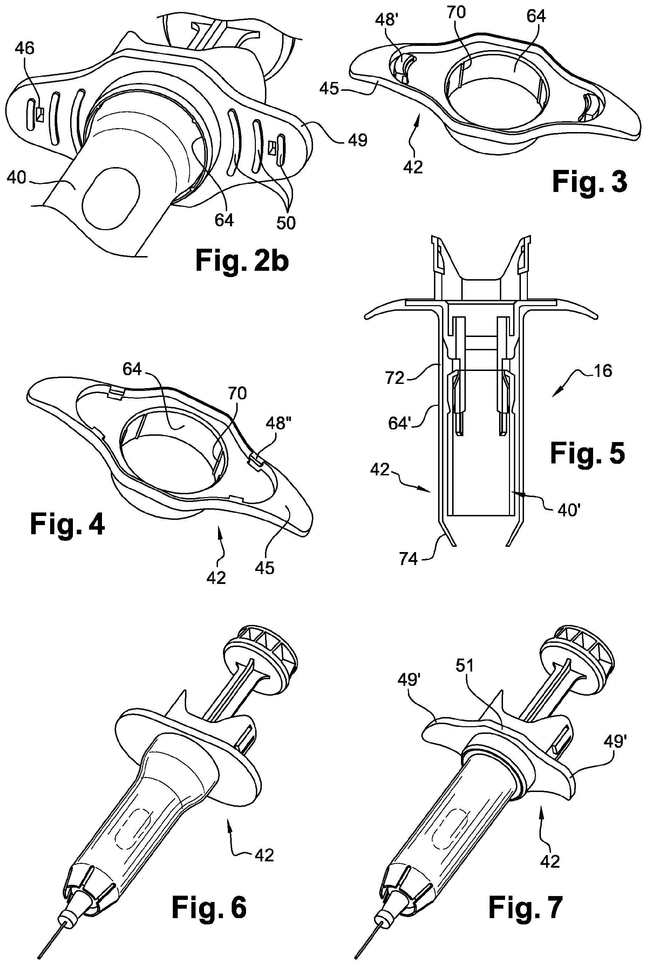

On a représenté sur les figures 1a, 1b, 2a et 2b un ensemble 10 d'un dispositif de sécurité 11 et d'une seringue d'injection 12. Le dispositif de sécurité 11 comprend un support de seringue 14, un fourreau de protection 16 et des moyens de rappel 18. Le fourreau 16 est monté coulissant par rapport au support 14 entre une position d'injection et une position de protection, sous l'action des moyens de rappel 18, composés ici d'un ressort en compression.

La seringue 12 est une seringue en verre, pré-remplie d'un produit. La seringue 12 est une seringue en verre standard, par exemple de 0,5 ou de 1 ml. Elle comprend un corps en verre, délimitant un réceptacle pour le produit et portant à son extrémité distale une aiguille d'injection 20, éventuellement par l'intermédiaire d'un luer lock. Lorsque le dispositif 11 est en position d'injection, l'aiguille 20 est découverte, c'est-à-dire qu'elle est visible par l'utilisateur, et lorsque le dispositif 11 est en position de protection, l'aiguille 20 est recouverte par le fourreau 16, de façon à empêcher une piqûre accidentelle après l'injection. Le corps de seringue en verre comprend par ailleurs sur son extrémité proximale une collerette 22, réalisée d'un seul bloc avec le corps de seringue. La collerette 22 permet en particulier de fixer le corps de la seringue 12 sur le support 14. La seringue 12 comprend par ailleurs un piston 24, comportant une base élastomère 26, une tige 28 et un poussoir 30. Le piston 24 est monté coulissant dans le corps de seringue de façon à pouvoir injecter le produit contenu dans le corps de seringue.

Le support de seringue 14 est une pièce en matière plastique de forme générale tubulaire. Il porte sur son extrémité proximale des moyens 32 d'encliquetage permanent de la collerette 22 de la seringue sur le support 14. Dans cet exemple, les moyens 22 comprennent des ergots répartis de façon angulaire autour de l'axe longitudinal X du dispositif et formant une butée dans la direction X de façon à immobiliser la collerette 22 entre les ergots et un siège circulaire réalisé sur le support 14. Le support 14 s'étend depuis l'extrémité proximale du corps de seringue 12 jusqu'au voisinage de son extrémité distale. Plus précisément, le support 14 laisse légèrement dépasser l'extrémité distale du corps de la seringue 12, comme on peut le voir sur la figure 1a. Le support 14 comprend également une butée 34 de retenue du support de seringue en position d'injection, ainsi qu'une butée 36 formant un siège pour le ressort 18. Les butées 34, 36 sont disposées sur l'extrémité proximale du support 14. Le support 14 comporte par ailleurs des moyens 38 d'immobilisation du support par rapport au fourreau 16 en position de protection, disposés sur l'extrémité distale du support 14. Plus précisément, les moyens 38 comprennent des ergots qui s'insèrent dans des fentes du fourreau 16 une fois le dispositif en position de protection, sous l'action du ressort, afin d'immobiliser le support 14 par rapport au fourreau 16. Le support 14 comprend également, sur une partie de sa surface extérieure tubulaire, des rainures s'étendant dans la direction longitudinale X et permettant de guider le coulissement du support 14 par rapport au fourreau 16 lors de l'assemblage des deux pièces et lors du déplacement du fourreau en position de protection.

Le fourreau de protection 16 est une pièce en matière plastique de forme générale tubulaire. Il comprend, dans cette invention, deux pièces rapportées l'une sur l'autre. Plus précisément, le fourreau 16 comprend une pièce 40, que l'on appelle dans la suite " fourreau initial " 40, et un organe 42 d'extension de la surface d'extension initiale. Le fourreau initial 40 est un fourreau qui, dans cet exemple, pourrait être utilisé seul en tant que fourreau du dispositif 11, l'organe 42 n'étant pas nécessaire à son fonctionnement. En particulier le fourreau initial 40 comporte une paroi de préhension 43, portant une surface 44 de préhension du dispositif 11 par un utilisateur, appelée surface de préhension initiale. Cette surface initiale 44 est une surface disposée du côté distal de la paroi 43, s'étend dans un plan radial, sensiblement perpendiculairement à l'axe longitudinal X, et comprend au moins deux parties diamétralement opposées, sous forme d'ailettes de préhension. La surface initiale 44 peut également prendre la forme d'une collerette circulaire faisant le tour du fourreau initial 40 et s'étendant dans un plan radial. La surface initiale 44 est configurée de telle sorte que, pour injecter le produit contenu dans la seringue 12, l'utilisateur agrippe deux de ses doigts, généralement l'index et le majeur, sous cette surface 44, de part et d'autre de l'axe X, et pose son pouce sur le poussoir 30 pour déplacer le piston 24. L'organe 42 permet d'adapter le dispositif 11 en vue d'une utilisation particulière, utilisation pour laquelle il est nécessaire d'avoir une surface de préhension plus grande qu'à l'accoutumée. A cet effet, l'organe 42 comporte une paroi 45 présentant du côté distal une surface 46 appelée surface de préhension agrandie, rapportée sur la surface initiale 44, de façon que le dispositif 11 présente une surface de préhension plus grande que la surface initiale 44. Dans cet exemple, la surface agrandie 46 recouvre totalement et agrandit la surface initiale 44. En effet comme on peut le constater sur les figures 1a, 1b, la surface 46 a une longueur dans la direction radiale qui est plus grande que celle de la surface initiale 44. L'organe 42 est fixé sur le fourreau initial 40 par des moyens d'encliquetage 48, comprenant ici des ergots 48 s'étendant à partir de la surface supérieure (ou surface proximale) de la paroi 45 portant la surface de préhension agrandie 46. Les moyens 48 permettent d'encliqueter l'organe 42 sur le fourreau initial 40, plus précisément sur la paroi de préhension initiale 43. On notera que la surface de préhension agrandie 46 présente des reliefs antidérapants 50. Par ailleurs, comme on peut le voir sur la figure 2a, la surface de préhension agrandie 46 présente deux extrémités radiales opposées 49, connectées par deux bords 51 présentant chacun un méplat et facilitant l'orientation du dispositif 11 pendant l'assemblage, le transport et le stockage.

Le fourreau de protection 16 comporte par ailleurs certaines fonctionnalités, qui sont portées par le fourreau initial 40 dans cet exemple.

Plus précisément, le fourreau comporte une extrémité distale 52 agencée de façon à empêcher que le doigt d'une personne soit en contact avec l'aiguille lorsque le fourreau 16 est en position de protection. L'extrémité 52 présente à cet effet une diminution de diamètre pour empêcher l'introduction d'un doigt à l'intérieur du fourreau 16. Dans cet exemple, l'extrémité 52 est fendue, de façon à présenter des "pétales", pour permettre le démoulage du fourreau initial 40 par élasticité des pétales. Le fourreau 16 comporte par ailleurs des moyens 54 de retenue du dispositif en position d'injection, les moyens 54 étant escamotables lorsque le piston arrive en fin de course, de façon à déclencher le déplacement en position de protection. Plus précisément, les moyens 54 comprennent des ergots disposés sur l'extrémité proximale du dispositif 11 et s'étendant dans la direction radiale vers l'intérieur, de façon à retenir les butées 34. De préférence, le fourreau 16 comprend deux ergots 54 diamétralement opposés, portés chacun par une patte élastique présentant une rampe 56 coopérant avec le piston 24 de façon que les ergots 54 soient écartés vers l'extérieur et ainsi ne retiennent plus les butées 34 lorsque le piston arrive en fin de course.

Par ailleurs, le fourreau 16 présente des moyens 58 d'immobilisation du fourreau en position de sécurité, coopérant avec les moyens 38 prévus sur le support 14. Les moyens 58 comprennent ici deux fentes diamétralement opposées, délimitées en particulier par deux pattes élastiques 60, à débattement radial de sorte que les ergots 38 peuvent les passer en force sous l'action du ressort pour venir s'encliqueter dans les fentes 58.

Le fourreau 16 comporte également une surface de butée 62 formant un siège pour l'extrémité distale du ressort 18. La surface 62 est définie par le fond d'une gorge de réception du ressort 18, assurant un centrage du ressort au cours de l'assemblage du dispositif 11. On notera que la paroi intérieure de la gorge 62 n'est pas annulaire, elle comprend deux parties diamétralement opposées, séparées par un espace laissant la possibilité aux rainures de guidages du support 14 de coulisser par rapport au fourreau 16.

Dans cet exemple, l'organe d'extension 42 comporte une jupe annulaire 64 faisant saillie de la surface de préhension agrandie 46, donc disposée du côté distal de l'organe 42. La jupe 64 assure un centrage de l'organe d'extension 42 sur le fourreau initial 40. En outre, la jupe annulaire 64 est une jupe de masquage du ressort 18 en configuration comprimée. En effet, le fourreau initial 40 et le support 14 sont de préférence réalisés en matériau transparent, alors que l'organe 42 est réalisé en matériau opaque, de telle sorte que la jupe 64 permet de cacher le ressort lorsqu'il est comprimé.

Le fonctionnement de l'ensemble 10 des figures 1a, 1b, 2a et 2b va à présent être décrit.

Lorsque l'utilisateur veut procéder à une injection, le dispositif est en position d'injection, c'est-à-dire que le fourreau 16 ne recouvre pas l'aiguille, comme représenté sur la figure 1a. En principe, l'aiguille est recouverte par un capuchon, que l'utilisateur retire pour commencer l'injection. Il introduit ensuite l'aiguille 20 dans son corps ou dans celui d'un patient et commence l'injection en appuyant sur le piston 24 avec son pouce et en agrippant deux de ses doigts sous la surface de préhension agrandie 46. Lorsque le produit est complètement injecté dans le corps du patient, le piston arrive en fin de course et l'extrémité inférieure du poussoir 30 coopère avec les rampes 56 de façon à écarter les ergots 54 et à libérer le ressort 18 pour qu'il puisse se détendre. En se décomprimant, le ressort 18 pousse sur la surface 36 du support de seringue 14, de telle sorte que le support 14, donc la seringue 12, coulissent par rapport au fourreau 16, en "remontant" par rapport au fourreau 16, jusqu'à atteindre une position de protection, représentée sur la figure 1b. Dans cette position de protection, l'aiguille 20 est complètement recouverte par le fourreau 16 et il n'est pas possible d'être en contact avec elle, du fait que l'on ne peut faire passer un doigt par l'extrémité 52. Par ailleurs, le dispositif 11 est verrouillé dans cette position de protection grâce aux moyens d'immobilisation 38, 58 qui empêchent le fourreau 16 de coulisser par rapport au support 14.

Un exemple d'assemblage du dispositif 11 comprend les étapes suivantes. Le dispositif 11 est tout d'abord assemblé et pré-monté sans la seringue 12, de telle sorte que la seringue peut être fabriquée indépendamment du dispositif 11. Ceci est particulièrement avantageux : le laboratoire qui fabrique le médicament à injecter peut ainsi remplir la seringue 12 et la rapporter lui-même dans le dispositif pré-monté 11, par une simple insertion de la seringue, sans avoir besoin d'utiliser une machine relativement complexe d'assemblage du dispositif 11. En effet, cet assemblage est assuré directement par le fabricant du dispositif 11, dans une autre usine. Le fabricant du dispositif 11 fabrique tout d'abord par injection les éléments 40, 42, 14. Puis, il rapporte le ressort 18 à l'intérieur du fourreau initial 40, par l'extrémité proximale du fourreau 40, en l'introduisant dans la gorge 62, et introduit ensuite le support 14 à l'intérieur du fourreau 40 et du ressort 18. En appuyant sur le support 14, le ressort 18 se comprime, jusqu'à venir encliqueter les butées 34 du support 14 avec les ergots 54 du fourreau 40. On obtient ainsi un dispositif avec un ressort pré-comprimé, muni d'une surface de préhension 44, relativement petite, utilisable tel quel. Pour que le dispositif 11 soit utilisable avec plus de confort, on peut venir rapporter l'organe 42 d'extension de la surface de préhension. Dans ce cas, on introduit l'organe 42 par l'extrémité distale du fourreau initial 40 et l'on vient encliqueter les moyens 48 sur la paroi de préhension initiale 43. On dispose ainsi du dispositif 11 complètement assemblé, avec le ressort 18 pré-comprimé, de telle sorte que le dispositif 11 peut-être transporté jusqu'à un laboratoire, dans lequel on peut assembler la seringue pré-remplie 12 dans le dispositif 11, par une simple insertion de la seringue par l'extrémité proximale du dispositif 11, de façon à venir encliqueter la collerette 22 de la seringue dans les éléments 32, sans avoir besoin de prévoir une machine d'assemblage devant comprimer le ressort.

D'autres modes de réalisation de l'organe 42, similaires à celui décrit ci-dessus, sont représentés sur les autres figures.

Sur la figure 3, les moyens d'encliquetage 48' ne font pas saillie de la paroi de préhension agrandie 45 mais sont logés à l'intérieur d'un évidement réalisé dans cette paroi 45, de façon à ne pas dépasser de la surface proximale de la paroi 45, dans un souci esthétique. Par ailleurs, on voit sur la figure 3 l'orifice intérieur de la jupe annulaire 64, qui présente des éléments en saillie 70, pour le calage de l'organe 42 autour du fourreau initial 40. Sur la figure 4, les moyens d'encliquetage 48" sont légèrement différents des moyens 48 de la figure 1a. Ils comprennent des ergots s'étendant radialement de la surface proximale de la paroi 45, sans dépasser de cette paroi dans la direction longitudinale X, ce qui les rend plus discrets que les ergots 48.

Sur la figure 5, la jupe annulaire 64' s'étend sur une plus grande distance que la jupe 64 dans la direction longitudinale. Elle peut ainsi servir de support plus grand pour une étiquette. En outre, et de façon particulièrement avantageuse, la jupe 64' recouvre les moyens 38, 58 d'immobilisation du fourreau 16 par rapport au support 14, ce qui permet de les masquer et de les rendre inaccessibles, donc d'empêcher la réactivation du dispositif 11. La jupe annulaire 64' s'étend dans la direction longitudinale X jusqu'à l'extrémité distale du dispositif 11, de façon à recouvrir toute l'aiguille 20 de la seringue lorsque le fourreau 16 est en position de protection. Plus précisément, la jupe annulaire 64' comprend une partie tubulaire principale 72 et une extrémité distale 74 dont le diamètre diminue par rapport à celui de la partie tubulaire principale 72, pour éviter l'introduction d'un doigt. Sur ce mode de réalisation, le fourreau initial 40' ne comporte pas l'extrémité 52 du fourreau initial 40 de la figure 1b, la fonction de diminution de diamètre étant assurée par la jupe 64'.

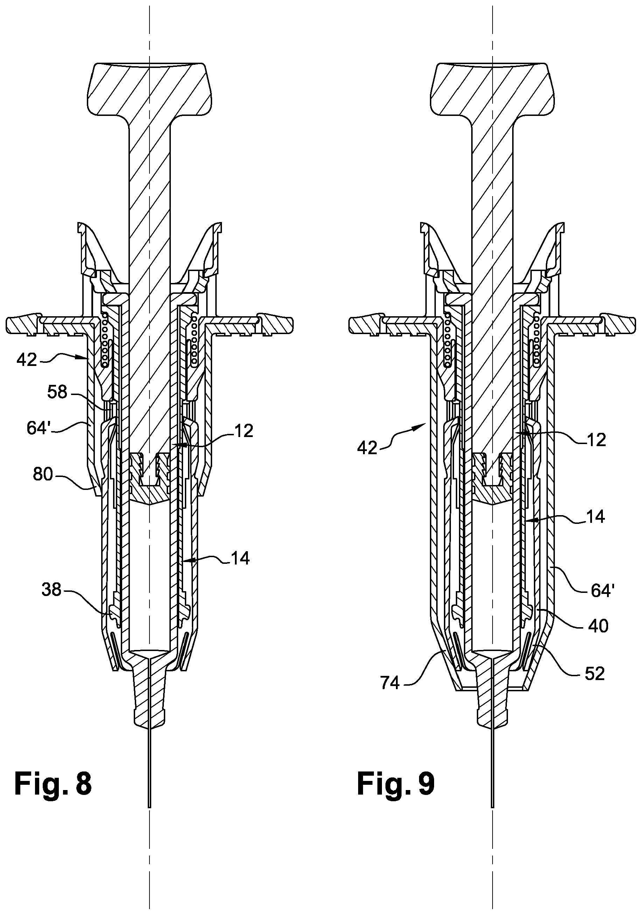

Sur la figure 9, la jupe annulaire 64' est sensiblement la même que celle de la figure 5, et le fourreau initial 40 est sensiblement le même que celui de la figure 1b. En particulier le fourreau initial 40 comporte l'extrémité tronconique 52 pour empêcher l'introduction d'un doigt. Cette extrémité 52 est recouverte par l'extrémité tronconique 74 de la jupe 64', d'où une protection encore meilleure de l'aiguille usagée.

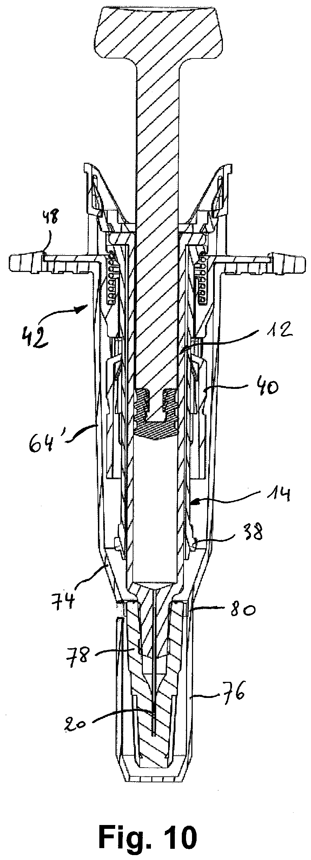

Sur la figure 10, le dispositif est sensiblement le même que celui de la figure 9. L'extrémité tronconique 74 de la jupe annulaire 64' est prolongée par un manchon fermé 76 qui recouvre l'aiguille 20, appelé manchon d'inviolabilité, fixé sur le dispositif avec un lien cassable de façon que l'utilisateur puisse vérifier que le dispositif n'a pas été ouvert avant l'injection. Plus précisément, l'organe d'extension 42 est moulé en une seule pièce avec le manchon d'inviolabilité 76, le manchon 76 étant relié à la jupe annulaire 64' par des pontets cassants 80 répartis sur la circonférence du manchon 76, entre le manchon 76 et l'extrémité tronconique 74 de la jupe annulaire 64'. L'aiguille 20 est en outre protégée par un capuchon amovible 78. En outre, les ergots 48 de fixation de l'organe 42 sur le fourreau initial 40 assurent une fixation inviolable de l'organe, c'est-à-dire qu'ils ne permettent pas, une fois l'organe 42 démonté du fourreau 40, de fixer à nouveau l'organe 42 sur le fourreau 40 : le démontage de l'organe 42 est irréversible.

Il est donc simple de vérifier, avant utilisation, que le dispositif n'a pas été ouvert. En effet, pour réaliser une injection, l'utilisateur casse les pontets 80 pour désolidariser le manchon 76 de la jupe 64' et ôte le capuchon 78 pour mettre l'aiguille 20 à nu. Une fois les pontets 80 cassés, il n'est pas possible de fixer à nouveau le manchon 76 sur la jupe annulaire 64'.

Sur la figure 8, la jupe annulaire 64' ne s'étend pas jusqu'à l'extrémité distale du dispositif de sécurité, à la différence des figures 5 et 9. Elle s'étend néanmoins sur une distance relativement grande autour du fourreau initial 40, suffisante pour recouvrir les moyens 38, 58 d'immobilisation du fourreau 16 par rapport au support 14, afin de les masquer et de les rendre inaccessibles. En outre la surface de la jupe 64' est suffisamment grande et lisse pour servir de support à une étiquette.

Sur la figure 6, l'organe d'extension 42 ne présente pas de jupe annulaire 64.

Sur la figure 7, la surface de préhension agrandie 46 présente deux extrémités radiales opposées 49', ces extrémités étant incurvées vers l'extrémité distale du dispositif 11.

On comprend que l'organe d'extension 42 présenté ci-dessus permet de fabriquer un dispositif de sécurité de taille sensiblement standard, comprenant les éléments 40, 18 et 14, tout en offrant la possibilité de l'adapter à une utilisation particulière, en ajoutant l'organe 42. On propose ainsi de "customiser" le dispositif de sécurité 11. En particulier on peut prévoir des organes d'extension 42 différents d'un dispositif 11 à un autre, réalisés dans une couleur, un matériau ou une forme différents.

On notera que l'invention n'est pas limitée aux modes de réalisation précédemment décrits. En particulier, les caractéristiques des différents modes de réalisation peuvent bien sûr être combinées les unes aux autres, ou encore dissociées.

## Revendications

1. Dispositif de sécurité (11) pour seringue d'injection (12), comprenant :
- un support de seringue (14),
- un fourreau (16) de protection d'une aiguille d'injection (20) portée par la seringue (12), le fourreau (16) étant monté coulissant par rapport au support de seringue (14) entre une position d'injection, dans laquelle l'aiguille (20) est découverte, et une position de protection, dans laquelle l'aiguille est recouverte, le fourreau (16) comportant une surface (44) de préhension du dispositif par un utilisateur, appelée surface de préhension initiale,
- un ressort (18) de rappel du fourreau en position de protection,
**caractérisé en ce que** le fourreau (16) comporte en outre un organe (42) d'extension de la surface de préhension initiale (44), rapporté sur le fourreau (16), de façon à présenter une surface de préhension (46) plus grande, dite surface de préhension agrandie.

2. Dispositif selon la revendication précédente, dans lequel l'organe d'extension (42) comporte une jupe annulaire (64, 64'), faisant saillie de la surface de préhension agrandie (46).

3. Dispositif selon la revendication précédente, dans lequel le ressort (18) est dans une configuration comprimée lorsque le fourreau (16) est en position d'injection, et dans lequel la jupe annulaire (64, 64') est une jupe de masquage du ressort (18) en configuration comprimée.

4. Dispositif selon la revendication 2 ou 3, dans lequel le fourreau (16) comporte des moyens (58) d'immobilisation du fourreau en position de protection, de préférence des moyens d'encliquetage, et la jupe annulaire (64') recouvre ces moyens (58) d'immobilisation du fourreau.

5. Dispositif selon l'une quelconque des revendications 2 à 4, dans lequel la jupe annulaire (64') s'étend dans la direction longitudinale jusqu'à l'extrémité distale du dispositif (11), de façon à recouvrir toute l'aiguille (20) de la seringue lorsque le fourreau (16) est en position de protection, la jupe annulaire (64') comprenant de préférence une partie tubulaire principale (72) et une extrémité distale (74) dont le diamètre diminue par rapport à celui de la partie tubulaire principale (72).

6. Dispositif selon l'une quelconque des revendications précédentes, dans lequel la surface de préhension agrandie (46) présente deux extrémités radiales opposées (49'), ces extrémités étant incurvées vers l'extrémité distale du dispositif.

7. Dispositif selon l'une quelconque des revendications précédentes, dans lequel la surface de préhension agrandie (46) présente des reliefs antidérapants (50).

8. Dispositif selon l'une quelconque des revendications précédentes, dans lequel la surface de préhension agrandie (46) présente deux extrémités radiales opposées (49), connectées par deux bords présentant chacun un méplat (51).

9. Dispositif selon l'une quelconque des revendications précédentes, comprenant un manchon d'inviolabilité (76), fixé sur le dispositif par un lien cassable, de préférence venu de moulage avec l'organe d'extension (42).

10. Ensemble d'un dispositif de sécurité (11) selon l'une quelconque des revendications 1 à 9 et d'une seringue pré-remplie (12).

11. Procédé d'assemblage d'un dispositif (11) selon l'une quelconque des revendications 1 à 9, au cours duquel on assemble tout d'abord une partie (40) du fourreau (16) et le support (14), puis l'on rapporte l'organe d'extension (42) sur cette partie (40) du fourreau (16).

## Patentansprüche

1. Sicherheitsvorrichtung (11) für eine Injektionsspritze (12), aufweisend
- eine Spritzenhalterung (14),
- eine Schutzhülle (16) für eine Injektionsnadel (20), die von der Spritze (12) getragen wird, wobei die Hülle (16) in Bezug auf die Spritzenhalterung (14) zwischen einer Injektionsposition, in der die Nadel (20) nicht bedeckt ist, und einer Schutzposition, in der die Nadel bedeckt ist, verschiebbar angebracht ist, wobei die Hülle (16) eine Fläche (44) zum Greifen der Vorrichtung durch einen Benutzer, die sogenannte Anfangsgreiffläche, aufweist,
- eine Feder (18) zum Zurückführen der Hülle in die Schutzposition, **dadurch gekennzeichnet, dass** die Hülle (16) außerdem ein Element (42) zum Verlängern der anfänglichen Greiffläche (44) aufweist, das an der Hülle (16) befestigt ist, um eine größere Greiffläche (46) aufzuweisen, die als vergrößerte Greiffläche bezeichnet wird.

2. Vorrichtung nach dem vorhergehenden Anspruch, wobei das Verlängerungselement (42) eine ringförmige Schürze (64, 64') aufweist, die von der vergrößerten Greiffläche (46) absteht.

3. Vorrichtung nach dem vorhergehenden Anspruch, wobei die Feder (18) in einer zusammengedrückten Einstellung ist, wenn sich die Hülle (16) in der Einspritzposition befindet, und wobei die ringförmige Schürze (64, 64') eine Schürze zum Abdecken der Feder (18) in der zusammengedrückten Einstellung ist.

4. Vorrichtung nach Anspruch 2 oder 3, bei der die Hülle (16) Mittel (58) zur Fixierung der Hülle in der Schutzstellung, vorzugsweise Schnappverschlussmittel, aufweist und die ringförmige Schürze (64') diese Mittel (58) zur Fixierung der Hülle überdeckt.

5. Vorrichtung nach einem der Ansprüche 2 bis 4, wobei sich die ringförmige Schürze (64') in Längsrichtung bis zum distalen Ende der Vorrichtung (11) erstreckt, so dass sie die gesamte Nadel (20) der Spritze abdeckt, wenn sich die Hülle (16) in der Schutzposition befindet, wobei die ringförmige Schürze (64') vorzugsweise einen rohrförmigen Hauptabschnitt (72) und ein distales Ende (74) umfasst, dessen Durchmesser relativ zu dem des rohrförmigen Hauptabschnitts (72) abnimmt.

6. Vorrichtung nach einem der vorangehenden Ansprüche, wobei die vergrößerte Greiffläche (46) zwei gegenüberliegende radiale Enden (49') aufweist, die zum distalen Ende der Vorrichtung hin gekrümmt sind.

7. Vorrichtung nach einem der vorhergehenden Ansprüche, wobei die vergrößerte Greiffläche (46) rutschfeste Reliefs (50) aufweist.

8. Vorrichtung nach einem der vorhergehenden Ansprüche, wobei die vergrößerte Greiffläche (46) zwei gegenüberliegende radiale Enden (49) aufweist, die durch zwei Kanten mit jeweils einer Abflachung (51) verbunden sind.

9. Vorrichtung nach einem der vorangehenden Ansprüche, umfassend eine manipulationssichere Hülle (76), die an der Vorrichtung durch ein zerbrechliches Glied befestigt ist, das vorzugsweise mit dem Verlängerungselement (42) geformt ist.

10. Anordnung aus einer Sicherheitsvorrichtung (11) nach einem der Ansprüche 1 bis 9 und einer vorgefüllten Spritze (12).

11. Verfahren zum Zusammenbau einer Vorrichtung (11) nach einem der Ansprüche 1 bis 9, bei dem zunächst ein Teil (40) der Hülle (16) und der Halterung (14) zusammengebaut werden und dann das Verlängerungselement (42) an diesem Teil (40) der Hülle (16) befestigt wird.

## Claims

1. Safety device (11) for an injection syringe (12), the safety device comprising:
· a syringe support (14);
· a protective sheath (16) for protecting an injection needle (20) carried by the syringe (12), the sheath (16) being mounted to slide relative to the syringe support (14) between an injection position in which the needle (20) is uncovered, and a protection position in which the needle is covered, the sheath (16) having a grip surface (44) enabling the device to be gripped by a user and referred to as the initial grip surface; and
· a return spring (18) for urging the sheath into the protection position;
the device being **characterized in that** the sheath (16) also includes an extender member (42) for extending the initial grip surface (44), fitted on the sheath (16), in such a manner as to present a larger grip surface (46), referred to as an enlarged grip surface.

2. Device according to the preceding claim, wherein the extender member (42) comprises an annular skirt (64, 64') projecting from the enlarged grip surface (46).

3. Device according to the preceding claim, wherein the spring (18) is in a compressed configuration when the sheath (16) is in the injection position, and wherein the annular skirt (64, 64') is a skirt for masking the spring (18) in the compressed configuration.

4. Device according to claim 2 or claim 3, wherein the sheath (16) includes means (58) for preventing the sheath from moving in the protection position, preferably snapfastener means, and the annular skirt (64') covers said means (58) for preventing the sheath from moving.

5. Device according to any one of claims 2 to 4, wherein the annular skirt (64') extends in the longitudinal direction to the distal end of the device (11), so as to cover all of the needle (20) of the syringe when the sheath (16) is in the protection position, the annular skirt (64') preferably comprising a main tubular portion (72) and a distal end (74) which diameter decreases relative to the diameter of the main tubular portion (72) .

6. Device according to any preceding claim, wherein the enlarged grip surface (46) presents two opposite radial ends (49'), these ends being curved towards the distal end of the device.

7. Device according to any preceding claim, wherein the enlarged grip surface (46) presents anti-skid portions (50) in relief.

8. Device according to any preceding claim, wherein the enlarged grip surface (46) presents two opposite radial ends (49) that are connected together by two sides, each presenting a flat surface (51).

9. Device according to any preceding claim, including a tamperproofing sleeve (76) fastened to the device by a breakable connection, preferably integrally molded with the extender member (42).

10. Assembly of a safety device (11) according to any one of claims 1 to 9 and a pre-filled syringe (12).

11. Method of assembling a device (11) according to any one of claims 1 to 9, wherein a portion (40) of the sheath (16) is initially assembled with the support (14), after which the extender member (42) is fitted on said portion (40) of the sheath (16).
